# EUROPEAN PATENT APPLICATION

(11) **EP 4 560 011 A1**
(43) Date of publication of application: **28.05.2025**
(21) Application number: 23842986.4
(22) Date of filing: 19.07.2023
(51) Int. Cl.: C12N 5/0775, A61K 35/28, A61K 35/32, A61L 27/36, A61L 27/38, A61L 27/40, A61P 43/00, C12M 3/00, C12N 15/12

(54) **METHOD FOR PRODUCING MESENCHYMAL STEM CELLS**

(30) Priority: 20.07.2022 JP 2022115178
(71) Applicant: FUJIFILM Corporation, Tokyo 106-8620 (JP)
(72) Inventor: OGURA, Izumi, Ashigarakami-gun, Kanagawa 258-8577 (JP); NAKAMURA, Kentaro, Ashigarakami-gun, Kanagawa 258-8577 (JP)
(74) Representative: Hoffmann Eitle
(86) International application number: PCT/JP2023/026332
(87) International publication number: WO 2024/019063

(57) **Abstract**

In the research and cell therapy of a mesenchymal stem cell, it is necessary to secure a large number of cells. An object of the present invention is to provide a production method of a mesenchymal stem cell, which enables a large amount of mesenchymal stem cells maintaining undifferentiated properties to be produced. According to the present invention, there is provided a production method of a mesenchymal stem cell, including culturing a mesenchymal stem cell in a culture vessel in which a water contact angle of a bottom surface that is brought into contact with a cell is 70° or more, and subculturing the mesenchymal stem cell two or more times.

## Description

### Technical Field

The present invention relates to a production method of a mesenchymal stem cell which maintains undifferentiated properties.

### Background Art

A mesenchymal stem cell is a somatic stem cell derived from mesodermal tissue (mesenchymal tissue), and has a differentiation potency into cells belonging to the mesenchyme. The mesenchymal stem cells are expected to be applied to regenerative medicine such as reconstruction of bone, blood vessels, or myocardium.

A culture vessel for culturing stem cells such as mesenchymal stem cells has been studied so far. For example, Patent Document 1 describes a vessel for culturing stem cells, which is a scaffold material for culturing stem cells, the vessel including a resin film containing a synthetic resin in at least a part of a culture region for stem cells. Patent Document 2 describes a culture method for stem cells, including proliferating stem cells adhering to a culture vessel in the culture vessel containing a liquid culture medium, peeling the proliferated stem cells from the culture vessel with a stripper that does not contain a proteolytic enzyme but contains at least a chelating agent, and suspending the peeled stem cells in the liquid culture medium, in which at least a portion of the culture vessel to which the stem cells adhere is formed of an alicyclic structure-containing polymer. The dish for adhering cells (manufactured by TrueLine; model number of TR4002; diameter of 10 cm; made of polystyrene) used in Comparative Example 1 of Patent Document 2 has a surface subjected to a corona treatment (hydrophilic treatment).

### Prior Art Documents

### Patent Documents

Patent Document 1: JP2019-115323A
Patent Document 2: JP2018-201403A

### SUMMARY OF THE INVENTION

### Object to be solved by the invention

In the research and cell therapy of a mesenchymal stem cell, it is necessary to secure a large number of cells. However, in a case where the culture period of the mesenchymal stem cells is prolonged to obtain a large amount of mesenchymal stem cells, there is an issue that the undifferentiated properties of the mesenchymal stem cells decrease with the passage of the culture period. An object to be achieved by the present invention is to provide a production method of a mesenchymal stem cell, which enables a large amount of mesenchymal stem cells maintaining undifferentiated properties to be produced.

### Means for solving the object

As a result of intensive studies to achieve the above object, the present inventors have found that, in a case where mesenchymal stem cells are cultured in a culture vessel in which a water contact angle of a bottom surface that is brought into contact with cells is 70° or more, the undifferentiated properties of the mesenchymal stem cells can be maintained even in a case where the mesenchymal stem cells are subcultured two or more times. The present invention has been completed based on the above findings.

According to the present invention, the following inventions are provided.
<1> A production method of a mesenchymal stem cell, comprising culturing a mesenchymal stem cell in a culture vessel in which a water contact angle of a bottom surface that is brought into contact with a cell is 70° or more, and subculturing the mesenchymal stem cell two or more times.
<2> The production method according to <1>, in which the culture vessel is a culture vessel having a volume of 0.6 L or more.
<3> The production method according to <2>, in which the culture vessel is a multi-layer flask.
<4> The production method according to any one of <1> to <3>, in which the mesenchymal stem cell is a synovial mesenchymal stem cell or a bone marrow mesenchymal stem cell.
<5> The production method according to any one of <1> to <3>, in which the culture vessel is made of polystyrene.
<6> The production method according to any one of <1> to <3>, in which the culturing is performed in absence of a ROCK inhibitor.
<7> The production method according to any one of <1> to <3>, in which the mesenchymal stem cell is subcultured five or more times.
<8> The production method according to any one of <1> to <3>, in which the culturing is performed until PDL, which is a population doubling level, after the start of the culturing, is 5 or more.
<9> The production method according to any one of <1> to <3>, in which in a case where an expression level of Meflin at a first subculturing is set to 1, a relative expression level of Meflin during a culture period is maintained at 0.40 or more.
<10> The production method according to any one of <1> to <3>, in which a mesenchymal stem cell to be produced is a cell for cell therapy.
<11> The production method according to <10>, in which the cell for cell therapy is an allogeneic cell for therapy.

### Effect of the invention

According to the production method of a mesenchymal stem cell according to the embodiment of the present invention, it is possible to produce a large amount of mesenchymal stem cells in which undifferentiated properties are maintained.

### BRIEF DESCRIPTION OF THE DRAWINGS

[Fig. 1]Fig. 1 shows a change in expression of Meflin over time in synovial MSC cultured in a vessel for cell culture (Comparative Example).
[Fig. 2]Fig. 2 shows a change in expression of Meflin over time in bone marrow MSC cultured in a vessel for cell culture (Comparative Example).
[Fig. 3] Fig. 3 shows cell proliferation of the synovial MSC over time cultured in a vessel for cell culture (Comparative Example).
[Fig. 4] Fig. 4 shows a change in expression of Meflin over time in synovial MSC cultured in culture vessel made of polystyrene (Example).
[Fig. 5] Fig. 5 shows a change in expression of Meflin over time in bone marrow MSC cultured in culture vessel made of polystyrene (Example).
[Fig. 6] Fig. 6 shows cell proliferation of the synovial MSC over time cultured in a culture vessel made of polystyrene (Example).

### EMBODIMENTS FOR CARRYING OUT THE INVENTION

Hereinafter, an example of the embodiment of the present disclosure will be described. However, the present disclosure is not limited to the following embodiments, and can be implemented with appropriate changes within the scope of the object of the present disclosure. In the present specification, the numerical range indicated by using "to" means a range including numerical values described before and after "to" as a minimum value and a maximum value, respectively.

The production method of a mesenchymal stem cell according to the embodiment of the present invention includes culturing a mesenchymal stem cell in a culture vessel in which a water contact angle of a bottom surface that is brought into contact with a cell is 70° or more, and subculturing the mesenchymal stem cell two or more times.

In the related art, for culturing mesenchymal stem cells, a culture vessel in which a culture surface is subjected to a hydrophilic treatment to increase cell adhesiveness is used. However, in the culture method for mesenchymal stem cells in the related art, the undifferentiated properties of cells cannot be maintained, and the expression level of an undifferentiated marker (Meflin) is halved in about 5 subculture. In the present invention, in a case where mesenchymal stem cells are cultured in a polystyrene culture vessel which has not been subjected to a surface treatment, the mesenchymal stem cells can be cultured without a decrease in the expression level of an undifferentiated marker (Meflin) even after a plurality of times of subculturing and long-term culturing, as in the related art. That is, it has been found that in the method according to the embodiment of the present invention, the culture can be performed by 5 or more subculture while maintaining the expression of the undifferentiated marker.

In addition, in JP2019-115323A, a ROCK inhibitor (Y27632) is added during culturing, but the method according to the embodiment of the present invention does not require a ROCK inhibitor.

As described above, according to the method of the present invention, it is possible to produce a large amount of mesenchymal stem cells in which undifferentiated properties are maintained. That is, according to the present invention, cells in which the undifferentiated properties are maintained for a long period of time can be cultured, and thus a large amount of cells for a cell therapy product can be easily produced. Therefore, the mesenchymal stem cells produced by the production method according to the embodiment of the present invention can be used in industries where a large amount of cells is required, such as cell therapy.

In addition, in general cell culture, a culture vessel with a coated surface is used, but in the present invention, by using an uncoated polystyrene culture vessel, the coating work is reduced, and culture can be performed at a low cost.

### <Mesenchymal stem cell>

The stem cell refers to an immature cell having self-replication ability and differentiation proliferation ability, and includes pluripotent stem cell, multipotent stem cell, unipotent stem cell, and the like, depending on the differentiation ability. The "stem cell" is generally defined as an undifferentiated cell having "self-renewal ability" capable of proliferating while maintaining an undifferentiated state and "differentiation pluripotency" capable of differentiating into all three germ layers. The pluripotent stem cell means a cell having an ability to differentiate into all tissues and cells constituting a living body. The multipotent stem cell means a cell that has an ability to differentiate into a plurality of types of tissues or cells, but not all types of tissues or cells. The unipotent stem cell means a cell having an ability to differentiate into a specific tissue or cell.

The mesenchymal stem cell broadly refers to a population of stem cells or progenitor cells thereof that can differentiate into all or some of mesenchymal cells such as an osteoblast, a chondroblast, and an adipoblast.

The origin of the mesenchymal stem cells is not particularly limited, and they may be cells of rodents such as a rat, a mouse, a hamster, and a guinea pig, the Leporidae such as a rabbit, the Ungulata such as a pig, a cow, a goat, and a sheep, the Carnivora such as a dog and a cat, and primates such as a human, a monkey, a rhesus monkey, a marmoset, an orangutan, or a chimpanzee. The mesenchymal stem cell is preferably a human mesenchymal stem cell.

In addition, the mesenchymal stem cell is preferably a cell derived from any of synovium, bone marrow, fat, umbilical cord, dental pulp, a fetus-derived composition, or an induced pluripotent stem cell. The mesenchymal stem cell is more preferably a synovial mesenchymal stem cell or a bone marrow mesenchymal stem cell.

### <Culture vessel>

In the present invention, mesenchymal stem cells are cultured in a culture vessel in which a water contact angle of a bottom surface that is brought into contact with cells is 70° or more.

To measure the water contact angle of the bottom surface of the culture vessel, which is brought into contact with the cells, the bottom surface of the culture vessel, which is brought into contact with the cells (in a case where the culture vessel is a well, the bottom surface of the well) is cut out to an appropriate size (for example, about 2 cm square) with an ultrasonic cutter or the like, a contact angle meter PG-X (MATSUBO Corporation) is set on the cut out base material, and the static contact angle is measured by the sessile drop method. The analysis is performed using PG software ver. 3.1 to obtain a measured value.

The water contact angle of the bottom surface that is brought into contact with the cells is only required to be 70° or more, and may be 75° or more, 80° or more, 85° or more, or 90° or more. The upper limit of the water contact angle of the bottom surface that is brought into contact with the cells is not particularly limited, but is generally 100° or less.

It is preferable that the bottom surface of the culture vessel, which is brought into contact with the cells, is not subjected to a hydrophilization treatment (for example, a corona treatment or the like). In a case where a hydrophilization treatment (for example, a corona treatment or the like) is performed, the water contact angle of the bottom surface that is brought into contact with the cells is generally less than 70°.

The material of the culture vessel is not particularly limited as long as the water contact angle of the bottom surface that is brought into contact with the cells can be set to 70° or more, and for example, polystyrene, polypropylene, polyethylene, or the like can be used, and among these, polystyrene is particularly preferable.

The culture vessel is preferably a culture vessel for large-scale culture, and for example, a culture vessel having a volume of 0.6 liters (L) or more. The capacity of the vessel may be 1.0 L or more, 10 L or more, 50 L or more, or 100 L or more. The upper limit of the capacity of the vessel is not particularly limited, but is generally 5,000 L or less.

The form of the culture vessel is not particularly limited as long as it can be used for culturing mesenchymal stem cells, and examples thereof include a flask (for example, may be a multi-layered flask), a flask for tissue culture, a dish, a petri dish, a dish for tissue culture, a multi-dish, a microplate, a micro-well plate, a multi-plate, a multi-well plate, a microslide, a chamber slide, a schale, a tube, a tray, a culture bag, a roller bottle, and the like.

The culture vessel may be cell-adhesive or cell-non-adhesive, and it is appropriately selected depending on the intended purpose.

### <Culture of mesenchymal stem cells>

The culture of mesenchymal stem cells can be performed using a culture medium suitable for culturing the mesenchymal stem cells.

As the culture medium suitable for culturing the mesenchymal stem cells, a culture medium used for culturing general animal cells can be used, and examples thereof include αMEM, a Dulbecco Modified Eagle Medium (DMEM), a mixed medium of DMEM and F12 (DMEM:F12 = 1:1), an RPMI medium (a GIBCO (registered trade name) RPMI 1640 medium or the like), and a mixed medium of DMEM/F12 and RPMI (DMEM/F12:RPMI = 1: 1), which are not particularly limited.

As a culture medium suitable for culturing mesenchymal stem cells, it is preferable to use the culture medium A described in Examples of JP2021-040551A.

The culture medium described in JP2021-040551A is a culture medium containing:
essential amino acids (histidine, isoleucine, leucine, lysine, methionine, phenylalanine, threonine, tryptophan, and valine);
non-essential amino acids (one or more selected from the group consisting of glycine, alanine, arginine, asparagine, aspartic acid, cysteine, cystine, glutamines, glutamic acid, proline, serine, and tyrosine);
biotin, vitamin B12, and at least one other vitamin;
inorganic salts (one or more selected from the group consisting of calcium chloride, magnesium sulfate, potassium chloride, sodium hydrogen carbonate, sodium chloride, and sodium dihydrogen phosphate);
at least one of sugars or pyruvates;
lipoic acid; and
ascorbic acid derivatives (ascorbic acid-2-phosphate, trisodium ascorbate-2-phosphate, magnesium ascorbate-2-phosphate, ascorbic acid-2-glycoside, and the like); and may further contain phenol red.

The culture medium may be a culture medium containing serum or may be a culture medium not containing serum. The lower limit of the amount of serum in the culture medium containing the serum is preferably 2% by volume or more, more preferably 5% by volume or more, and still more preferably 10% by volume or more. The upper limit value thereof is generally 20% by volume or less.

Examples of the serum include animal-derived serum. Although not particularly limited, the serum is preferably serum derived from a mammal (for example, bovine fetal serum, human serum, and the like).

The culture medium may contain a serum substitute. The serum substitute includes a proliferation factor, but other examples thereof include albumin, albumin substitutes such as lipid-rich albumin and recombinant albumin, plant starch, dextran, protein hydrolysate, transferrin or other iron transporters, fatty acid, insulin, collagen precursor, trace elements, 2-mercaptoethanol, 3'-thioglycerol, and equivalents thereof. Specific examples of the serum substitute include those prepared by the method described in WO98/30679A, and commercially available knockout Serum Replacement (KSR International Inc.), Chemically-defined Lipid concentrated (Life Technologies Corporation), and Glutamax (Life Technologies Corporation).

The culture medium may further contain a proliferation factor. Examples of the proliferation factor include a basic fibroblast growth factor.

The culture medium may further contain an antibiotic. Examples of the antibiotic include streptomycin, gentamicin (gentamicin sulfate and the like), and penicillin.

The culture medium can contain a known additive, as necessary. Examples of the additive include polyamines (for example, putrescine and the like), a reducing agent (for example, 2-mercaptoethanol or the like), and a buffering agent (for example, 4-(2-hydroxyethyl)-1-piperazineethanesulfonic acid (HEPES)).

The culture conditions for the mesenchymal stem cells can be appropriately set. For example, the culture temperature is not particularly limited, but is about 30°C to 40°C, and preferably about 37°C. The CO₂ concentration is generally about 1% to 10% and preferably about 2% to 5%.

In the present invention, the culture of the mesenchymal stem cell can be performed in the absence of a Rho-associated coiled-coil forming kinase (Rho binding kinase) inhibitor (ROCK inhibitor).

### <Subculture>

In the present invention, the mesenchymal stem cells are subcultured two or more times. The number of times of the subculture is not particularly limited as long as it is 2 or more, and may be 3 or more, 4 or more, 5 or more, 6 or more, 7 or more, 8 or more, 9 or more, 10 or more, 12 or more, 14 or more, or 16 or more. The upper limit of the number of times of the subculture is not particularly limited, but is generally 100 times or less, and may be 50 times or less.

In the present invention, by culturing the mesenchymal stem cells after performing the subculture twice or more as described above, it is possible to culture the mesenchymal stem cells for a long period of time (10 days or more, 14 days or more, 20 days or more, 25 days or more, 30 days or more, 35 days or more, 40 days or more, or 50 days or more) while maintaining the undifferentiated properties of the mesenchymal stem cells, thereby it is possible to obtain a large amount of mesenchymal stem cells.

The subculture of the mesenchymal stem cells can be performed by a general method. Specifically, after culturing the cells, the culture supernatant is aspirated, and the culture surface is washed with an appropriate buffer solution. Thereafter, the treatment with an enzyme for cell peeling such as trypsin (for example, TrypLE (registered trademark) Express (Gibco)) is performed. The enzymatic treatment can be performed, for example, at 30°C to 45°C, preferably 35°C to 40°C, and as an example, 37°C for 1 minute to 60 minutes, preferably 1 minute to 20 minutes, and more preferably 2 minutes to 10 minutes. After the enzymatic treatment, the cells are neutralized with a culture medium, and then the cells are collected and centrifuged, and the supernatant can be aspirated. As necessary, the obtained cells may be counted, and then the cells may be seeded in a culture vessel containing a culture medium at an appropriate cell density. The cell density is, for example, 0.01 to 3.0 × 10⁴ cells/cm², preferably 0.05 to 1.0 × 10⁴ cells/cm², more preferably 0.10 to 0.5 × 10⁴ cells/cm², and particularly preferably 0.15 to 0.3 × 10⁴ cells/cm².

### <Population doubling level and expression level of Meflin>

In the present invention, the culture can be performed until the PDL, which is a population doubling level after the start of the culturing, is preferably 5 or more. The culture can be performed until the PDL, which is a population doubling level after the start of culturing, is more preferably 10 or more, 15 or more, 20 or more, or 25 or more. The upper limit of the PDL is not particularly limited, but is generally 200 or less, and may be 100 or less. The population doubling level (PDL) is a population doubling value, and represents the number of times that a cell population has divided so far.

In the present invention, in a case where the expression level of Meflin at the first subculture is set to 1, the relative expression level of Meflin during the culture period is preferably maintained at 0.40 or more. The maintenance of the relative expression level of Mefflin, which is a marker of the undifferentiated properties of mesenchymal stem cells, at 0.40 or more means that the undifferentiated properties of mesenchymal stem cells is maintained. In the present invention, in a case where the expression level of Meflin at the first subculture is set to 1, the relative expression level of Meflin during the culture period is more preferably maintained at 0.50 or more. The upper limit of the relative expression level of Meflin is not particularly limited, but is generally 10 or less, and may be 5 or less, 3 or less, or 2 or less.

A measuring method of the relative expression level of Meflin is not particularly limited, and examples thereof include a method of extracting RNA from cells, synthesizing cDNA, and performing Real-Time PCR using the synthesized cDNA, a probe of 18S or Meflin, and a PCR reagent.

### <Produced mesenchymal stem cells>

The use of the mesenchymal stem cells produced by the method according to the embodiment of the present invention is not particularly limited, but the cells can be used for medical use as a cell for cell therapy, as an example. The mesenchymal stem cells can be used as they are in an undifferentiated state or after being differentiated into bone cells, cartilage cells, adipocytes, or the like, depending on the target disease or the like. Examples of the target disease include aseptic necrosis of lunate bone, aseptic necrosis of femoral head, intractable osteochondritis, lumbar intervertebral disc herniation, ischemic heart disease, epidermal bullous disease, and the like. In addition, the mesenchymal stem cells can also be used for cosmetic surgery after differentiation into adipocytes.

The cell for cell therapy may be either an allogeneic cell for medical treatment or an autologous cell for medical treatment, but is preferably an allogeneic cell for medical treatment.

Furthermore, in the production method according to the embodiment of the present invention, a cell culture supernatant can also be obtained. The cell culture supernatant contains various cytokines (proteins) secreted from the mesenchymal stem cells depending on the type of the mesenchymal stem cells, and can be applied to treatment or diagnosis. The extracellular vesicles can be extracted from the cell culture supernatant. Examples of the extracellular vesicle includes a substance secreted or the like from cultured cells. For example, exosomes secreted from cells, microvesicles obtained by being separated as a part of cells, apoptotic bodies obtained from died cells, and the like. These extracellular vesicles can also be used for the purpose of treatment and diagnosis, and can be used, for example, for cell therapy using the intercellular information transmission function carried out by the exosome, and the like.

The present invention will be further specifically described with reference to Examples; however, the present invention is not limited by Examples.

### Examples

### <Materials and Methods>

### (1) Acquisition of cells

As human synovial mesenchymal stem cells (human synovial MSC), a commercially available product from Articular Engineering, LLC (CCD-H-2910-N) was used.

As human bone marrow mesenchymal stem cells (human bone marrow MSC), a commercially available product (PT-2501) from Lonza Group AG was used.

### (2) Culture of mesenchymal stem cell (MSC)

The frozen MSCs were thawed, seeded in a vessel for cell culture (Falcon; #353046) or a polystyrene culture vessel (Falcon; #351146) at 0.15 to 0.3 × 10⁴ cells/cm², and cultured in an incubator at 37°C and in a 5% CO₂ atmosphere for 3 to 4 days.

The water contact angles of the vessel for cell culture and the polystyrene culture vessel were measured by the following method. The well bottom surfaces of the vessel for cell culture and the polystyrene container were cut out in a square of about 2 cm square with an ultrasonic cutter. A contact angle meter PG-X (MATSUBO Corporation) was set on the cut out base material, and a static contact angle was measured by a sessile drop method. The analysis was performed using PG software ver. 3.1 to obtain a measured value. As a result, the water contact angle of the vessel for cell culture was 45°, and the water contact angle of the polystyrene culture vessel was 80°.

As a culture medium, a culture medium obtained by adding fetal bovine serum (Gibco) (final concentration of 20%) and Antibiotic-Antimycotic (Gibco) (final concentration of 1%) to αMEM (culture medium A described in Examples of JP2021-040551A) was used.

After the culture, the culture supernatant was aspirated, the culture surface was washed twice with DPSB (Gibco), and a peeling treatment with TrypLE (registered trademark) Express (Gibco) was performed at 37°C for 5 minutes. After neutralization with a culture medium, the cells that had been peeled were collected and centrifuged, and the supernatant was aspirated. The cells were loosened by tapping, a part of the cell suspension obtained by adding a small amount of the culture medium thereto was taken out, and the number of cells was counted. The cells were calculated from the result, and the cells were seeded in a newly prepared vessel for cell culture (Falcon; #353046) or a polystyrene culture vessel (Falcon; #351146), containing a culture medium, at 0.15 to 0.3 × 10⁴ cells/cm² and the cells were continuously subcultured. In addition, the cells remaining after the subculture were collected for evaluating the expression level of the undifferentiated marker and stored at -80°C.

### (3) Evaluation of expression level of undifferentiated marker (Meflin)

RNA was extracted from the cells collected during the subculture using an RNeasy Mini Kit (QIAGEN), and cDNA was synthesized using a High Capacity RNA-to-cDNA kit (Applied Biosystems).

The synthesized cDNA, a probe of 18S or Meflin (ISLR), and TaqMan Gene Expression Master Mix (both Applied Biosystems) were mixed and subjected to Real-Time PCR.

The analysis was performed by the ΔΔCt method.

The expression level of the cells collected during the first subculture was set to 1, and the expression level within a range of 0.4 times to 2 times was set as the range of expression maintenance.

### (4) Evaluation of proliferation ability

The proliferation ability of MSC was evaluated by calculating a population doubling level (PDL) from the cell count at the time of the collection. Specifically, a part of the cell suspension was taken out, mixed with an equal amount of a trypan blue solution (FUJIFILM Wako Pure Chemical Corporation), injected into a blood cell counter, and counted by visual observation using a microscope.

The population doubling level (PDL) represents the number of times cells have divided from seeding to collection.

In a case where the population doubling level maintains linearity with respect to the subculture number, it can be determined that the proliferative ability is maintained. In a case where the population doubling level for each subculture is close to zero and an increase in the population doubling level is not observed, it can be determined that the proliferative ability is insufficient.

### <Result>

### (1) Comparative Example in which culture is performed in vessel for cell culture (Comparative Example)

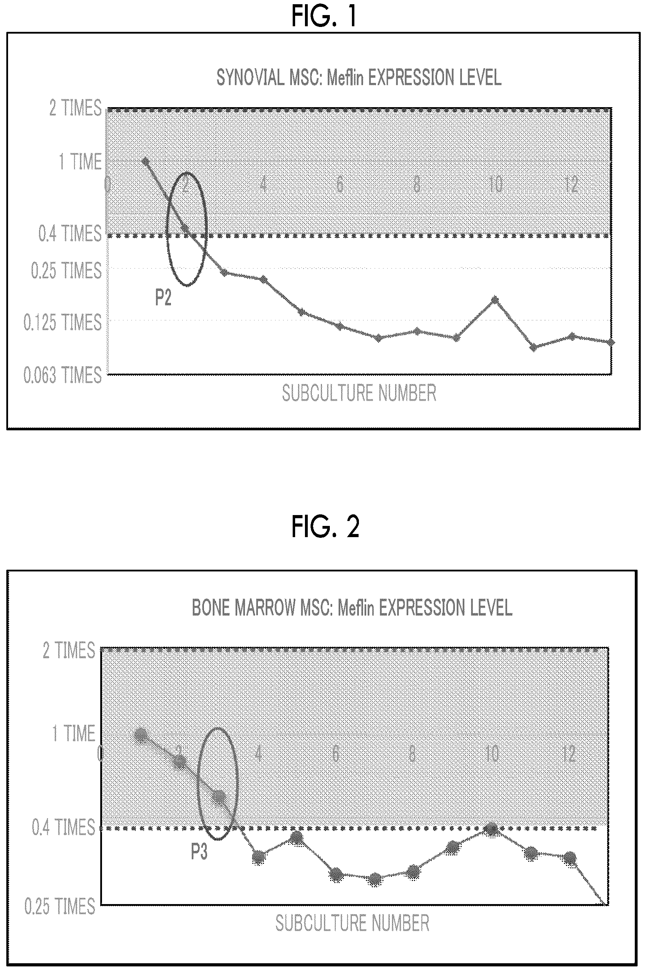

Fig. 1 shows a change in expression of Meflin over time in synovial MSC cultured in a vessel for cell culture (Comparative Example).

Fig. 2 shows a change in expression of Meflin over time in bone marrow MSC cultured in a vessel for cell culture (Comparative Example).

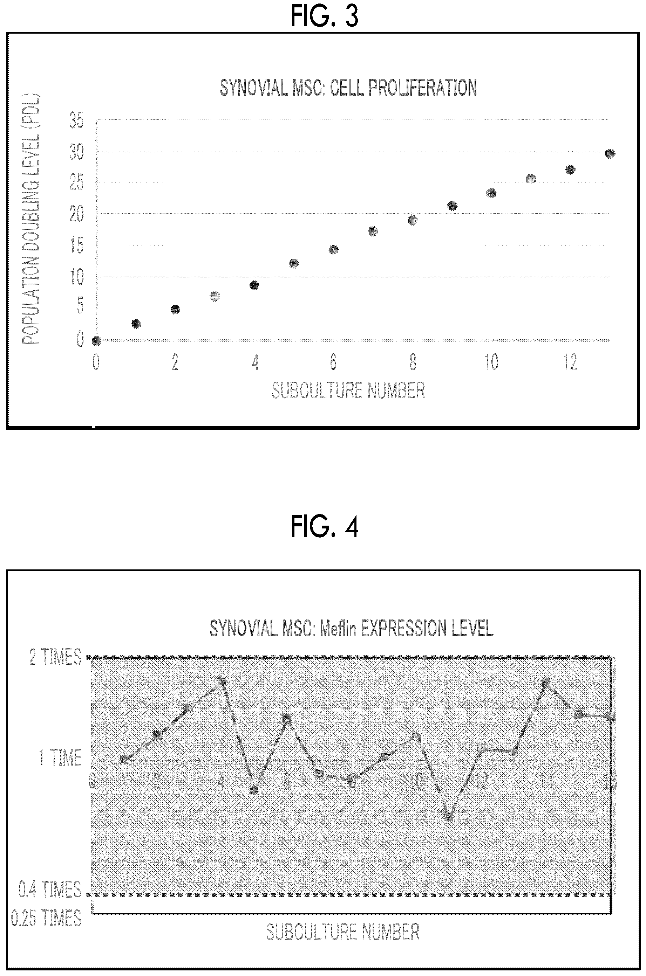

Fig. 3 shows cell proliferation of the synovial MSC over time cultured in a vessel for cell culture (Comparative Example).

In a case where the vessel for cell culture was used, the subculture number in a state where the expression of Meflin for the synovial MSC was maintained was 2, and the expression of Meflin was not maintained after the subculture number reached 3 or more. In addition, the subculture number in a state where the expression of Meflin in the bone marrow MSC was maintained was 3, and the expression of Meflin was not maintained after the subculture number reached 4 or more. In addition, the population doubling level (PDL) of the synovial MSC after the second subculture was 4.9.

### (2) Example in which culture is performed in polystyrene culture vessel (Example)

Fig. 4 shows a change in expression of Meflin over time in the synovial MSC cultured in a polystyrene culture vessel (Example).

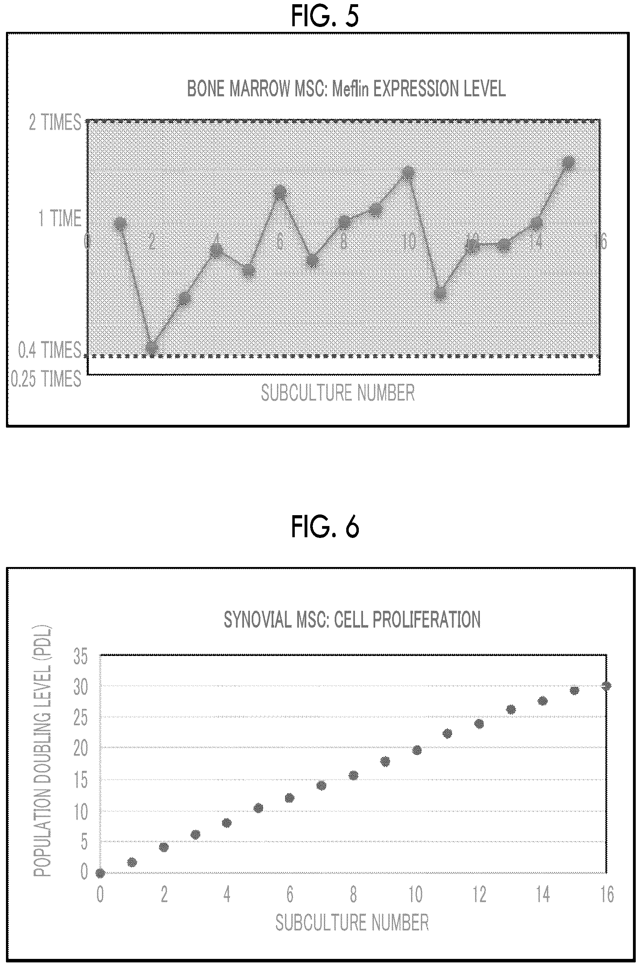

Fig. 5 shows a change in expression of Meflin over time in the bone marrow MSC cultured in a polystyrene culture vessel (Example). The expression level of Meflin after the second subculture of the bone marrow MSC was 0.43.

Fig. 6 shows cell proliferation of the synovial MSC over time cultured in a culture vessel made of polystyrene (Example).

In a case where a polystyrene culture vessel was used, the synovial MSC could be cultured up to the 16th subculture in a state where the expression of Meflin was maintained. In the case of the synovial MSC, the population doubling level (PDL) after 16th subculture was 30.0. In addition, in a case where a polystyrene culture vessel was used, the bone marrow MSC could be cultured up to the 15th subculture in a state where the expression of Meflin was maintained.

### (3) Conclusion

From the above results, it was found that in the culture in the polystyrene culture vessel, the subculture number can be increased while maintaining the undifferentiated properties, and the culture can be performed for a long period of time, as compared with the conventional method (culture vessel for cell culture).

## Claims

1. A production method of a mesenchymal stem cell, comprising:
culturing a mesenchymal stem cell in a culture vessel in which a water contact angle of a bottom surface that is brought into contact with a cell is 70° or more; and
subculturing the mesenchymal stem cell two or more times.

2. The production method according to claim 1,
wherein the culture vessel is a culture vessel having a volume of 0.6 L or more.

3. The production method according to claim 2,
wherein the culture vessel is a multi-layer flask.

4. The production method according to any one of claims 1 to 3,
wherein the mesenchymal stem cell is a synovial mesenchymal stem cell or a bone marrow mesenchymal stem cell.

5. The production method according to any one of claims 1 to 3,
wherein the culture vessel is made of polystyrene.

6. The production method according to any one of claims 1 to 3,
wherein the culturing is performed in absence of a ROCK inhibitor.

7. The production method according to any one of claims 1 to 3,
wherein the mesenchymal stem cell is subcultured five or more times.

8. The production method according to any one of claims 1 to 3,
wherein the culturing is performed until PDL, which is a population doubling level, after the start of the culturing, is 5 or more.

9. The production method according to any one of claims 1 to 3,
wherein in a case where an expression level of Meflin at a first subculturing is set to 1, a relative expression level of Meflin during a culture period is maintained at 0.40 or more.

10. The production method according to any one of claims 1 to 3,
wherein a mesenchymal stem cell to be produced is a cell for cell therapy.

11. The production method according to claim 10,
wherein the cell for cell therapy is an allogeneic cell for therapy.
